# EUROPEAN PATENT SPECIFICATION

(11) **EP 0 888 120 B1**
(45) Date of publication and mention of the grant of the patent: **19.11.2008**
(21) Application number: 97916104.9
(22) Date of filing: 21.03.1997
(51) Int. Cl.: C07K 7/00, A61K 38/00, A61K 38/08, A61P 31/18

(54) **IMMUNOGENIC HLA BINDING PEPTIDE AND ITS USES TO TREAT HIV INFECTION**
IMMUNOGENES HLA-BINDENDES PEPTID UND VERWENDUNG DESSELBEN ZUR HIV-INFEKTIONSBEHANDLUNG
PEPTIDE DE LIAISON AUX HLA ET SES UTILISATIONS POUR LE TRAITEMENT D'UNE INFECTION AU VIH

(30) Priority: 21.03.1996 US 13833 P; 20.03.1997 US 821739
(43) Date of publication of application: 07.01.1999
(60) Divisional application: 07075412.2
(73) Proprietor: Pharmexa Inc., San Diego, CA 92121 (US)
(72) Inventor: KUBO, Ralph, T., San Diego, CA 92130 (US); GREY, Howard, M., La Jolla, CA 92037 (US); SETTE, Alessandro, La Jolla, CA 92037 (US); CELIS, Esteban, San Diego, CA 92130 (US)
(74) Representative: Arends, William Gerrit
(86) International application number: PCT/US1997/004451
(87) International publication number: WO 1997/034617

(56) References cited:
- EP-A- 0 728 764
- WO-A-94/05304
- WO-A-94/20127
- WO-A-95/23234
- WO-A-97/04802
- CHEMICAL ABSTRACTS, vol. 125, no. 19, 4 November 1996 (1996-11-04) Columbus, Ohio, US; abstract no. 245055, P SHANKAR ET AL.: "Three regions of HIV-1 gp160 contain clusters of immunodominant CTL epitopes" XP002122110 & IMMUNOLOGY LETTERS., vol. 52, no. 1, January 1996 (1996-01), pages 23-30, AMSTERDAM., NL ISSN: 0165-2478
- CURR. OPIN. IMMUNOLOGY, 1994, Volume 6, ENGELHARD V.H., "Structure of Peptides Associated with MHC Class I Molecules", pages 13-23.
- J. IMMUNOLOGY, 11 December 1992, Volume 149, PARKER K.C. et al., "Sequence Motifs Important for Peptide Binding to the Human MHC Class I Molecule, HLA-A2", pages 3580-3587.
- ANNU. REV. IMMUNOL., 1993, Volume 11, RAMMENSEE H. et al., "Peptides Naturally Presented by MHC Class I Molecules", pages 213-244.
- MOL. IMMUNOLOGY, Volume 31, No. 8, December 1994, CELIS E. et al., "Identification of Potential CTL Epitopes of Tumor-Associated Antigen MAGE-1 for Five Common HLA-A Alleles", pages 1423-1430.

## Description

The present application is related to USSN 60/013,833, which is related to USSN 08/589,107, and USSN 08/451,913 and to USSN 081347,610, which is a continuation in part of USSN 08/159,339, which is continuation in part of USSN 08/103,396 which is a continuation in part of USSN 08/027,746 which is a continuation in part of USSN 07/926,666. It is also related to USSN 08/186,266.

### BACKGROUND OF THE INVENTION

The present invention relates to an immunogenic peptide consisting of SEQ ID NO: 101 and its use for the preparation of a medicament for treating HIV infection. The peptide is capable of binding selected major histocompatibility complex (MHC) molecules and inducing an immune response.

MHC molecules are classified as either Class I or Class II molecules. Class II MHC molecules are expressed primarily on cells involved in initiating and sustaining immune responses, such as T lymphocytes, B lymphocytes, macrophage, etc. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by cytotoxic T lymphocytes (CTLs), which then destroy the antigen-bearing cells. CTLs are particularly important in tumor rejection and in fighting viral infections. The CTL recognizes the antigen in the form of a peptide fragment bound to the MHC class I molecules rather than the intact foreign antigen itself. The antigen must normally be endogenously synthesized by the cell, and a portion of the protein antigen is degraded into small peptide fragments in the cytoplasm. Some of these small peptides translocate into a pre-Golgi compartment and interact with class I heavy chains to facilitate proper folding and association with the subunit β2 microglobulin. The peptide-MHC class I complex is then routed to the cell surface for expression and potential recognition by specific CTLs.

Investigations of the crystal structure of the human MHC class I molecule, HLA-A2.1, indicate that a peptide binding groove is created by the folding of the α1 and α2 domains of the class 1 heavy chain (Bjorkman et al., Nature 329:506 (1987). In these investigations, however, the identity of peptides bound to the groove was not determined.

Buus et al., Science 242:1065 (1988) first described a method for acid elution of bound peptides from MHC. Subsequently, Rammensee and his coworkers (Falk et al., Nature 351:290 (1991) have developed an approach to characterize naturally processed peptides bound to class I molecules. Other investigators have successfully achieved direct amino acid sequencing of the more abundant peptides in various HPLC fractions by conventional automated sequencing of peptides eluted from class I molecules of the B type (Jardetzky, et al., Nature 353:326 (1991) and of the A2.1 type by mass spectrometry (Hunt, et al., Science 225:1261 (1992). A review of the characterization of naturally processed peptides in MHC Class I has been presented by Rötzschke and Falk (Rötzschke and Falk, Immunol. Today 12:447 (1991).

Sette et al., Proc. Natl._Acad. Sci. USA 86:3296 (1989) showed that MHC allele specific motifs could be used to predict MHC binding capacity. Schaeffer et al., Proc. Natl. Acad. Sci. USA 86:4649 (1989) showed that MHC binding was related to immunogenicity. Several authors (De Bruijn et al., Eur. J. Immunol., 21:2963-2970 (1991); Pamer et al., 991 Nature 353:852-955 (1991)) have provided preliminary evidence that class I binding motifs can be applied to the identification of potential immunogenic peptides in animal models. Class I motifs specific for a number of human alleles of a given class I isotype have yet to be described. It is desirable that the combined frequencies of these different alleles should be high enough to cover a large fraction or perhaps the majority of the human outbred population. WO94/20127A is directed to the process of identification of peptides that bind specifically to HLA-A2.1.

Celis t et al Mol. Immunology, Vol. 31, No. 8, Dec. 1994, "Identification of Potential CTL Epitopes of Tumor-Associated MAGE-1 for five common HLA-A Alleles" is directed to the identification of CTL epitopes from the Tumor-associated MAGE-1 gene only.

CA 2173138 discloses immunogenic peptides derived from the whole protein of HIV of 8 to 11 amino acid residues. This peptide corresponds to an HLA binding motif.

Kubo et al "Definition of Specific Peptide Motifs for four Major HLA-A Alleles", Vol. 152, April 1994 is directed to the characterization of human HLA-Al, A3, A11 and A24 allele-specific CTL peptide motifs.

Despite the developments in the art, the prior art has yet to provide a useful human peptide-based vaccine or therapeutic agent based on this work. The present invention provides these and other advantages.

### SUMMARY OF THE INVENTION

The present invention relates to an immunogenic peptide consisting of SEQ ID NO:101 having binding motifs for MHC Class I molecules. The immunogenic peptide is 10 residues long and comprises conserved residues involved in binding proteins encoded by the appropriate MHC allele. A number of allele specific motifs have been identified.

For instance, the motif for HLA-A3.2 comprises from the N-terminus to C-terminus a first conserved residue of L, M, I, V, S, A, T and F at position 2 and a second conserved residue of K, R or Y at the C-terminal end. Other first conserved residues are C, G or D and alternatively E. Other second conserved residues are H or F. The first and second conserved residues are preferably separated by 6 to 7 residues.

The motif for HLA-A1 comprises from the N-terminus to the C-terminus a first conserved residue of T, S or M, a second conserved residue of D or E, and a third conserved residue of Y. Other second conserved residues are A, S or T. The first and second conserved residues are adjacent and are preferably separated from the third conserved residue by 6 to 7 residues. A second motif consists of a first conserved residue of E or D and a second conserved residue of Y where the first and second conserved residues are separated by 5 to 6 residues.

The motif for HLA-A11 comprises from the N-terminus to the C-terminus a first conserved residue of T or V at position 2 and a C-terminal conserved residue of K. The first and second conserved residues are preferably separated by 6 or 7 residues.

The motif for HLA-A24.1 comprises from the N-terminus to the C-terminus a first conserved residue of Y, F or W at position 2 and a C terminal conserved residue of F, I, W, M or L. The first and second conserved residues are preferably separated by 6 to 7 residues.

Epitopes on a number of potential target proteins have been identified in this manner. The peptides were prepared based on sequences of antigenic proteins from pathogens (*e.g*., viral pathogens, fungal pathogens, bacterial pathogens, protozoal pathogens, and the like) or from antigens associated with cancer. Examples of suitable antigens include prostate specific antigen (PSA), hepatitis B core and surface antigens (HBVc, HBVs) hepatitis C antigens, malignant melanoma antigen (MAGE-1) Epstein-Barr virus antigens, human immunodeficiency type-1 virus (HIV1) and papilloma virus antigens. The peptide of SEQ ID NO: 101 or nucleic acids that encode it are useful in pharmaceutical compositions for both in vivo and ex vivo therapeutic applications.

### Definitions

The term "peptide" is used in the present specification to designate a series of residues, typically L-amino acids, connected one to the other by peptide bonds between the alpha-amino and carbonyl groups of adjacent amino acids. The peptide of the invention is 10 residues in length.

An "immunogenic peptide" is a peptide which comprises an allele-specific motif such that the peptide will bind the MHC allele and be capable of inducing a CTL response. Thus, immunogenic peptides are capable of binding to an appropriate class I MHC molecule and inducing a cytotoxic T cell response against the antigen from which the immunogenic peptide is derived.

The relationship between binding affinity for MHC class I molecules and immunogenicity of discrete peptide epitopes has been analyzed in two different experimental approaches (Sette, et al., J. Immunol., 153:5586-5592 (1994)). In the first approach, the immunogenicity of potential epitopes ranging in MHC binding affinity over a 10,000-fold range was analyzed in HLA-A*0201 transgenic mice. In the second approach, the antigenicity of approximately 100 different hepatitis B virus (HBV)-derived potential epitopes, all carrying A*0201 binding motifs, was assessed by using PBL of acute hepatitis patients. In both cases, it was found that an affinity threshold of approximately 500 nM (preferably 500 nM or less) determines the capacity of a peptide epitope to elicit a CTL response. These data correlate well with class I binding affinity measurements of either naturally processed peptides or previously described T cell epitopes. These data indicate the important role of determinant selection in the shaping of T cell responses.

A "conserved residue" is an amino acid which occurs in a significantly higher frequency than would be expected by random distribution at a particular position in a peptide motif. Typically a conserved residue is one at which the immunogenic peptide may provide a contact point with the MHC molecule. One to three, preferably two, conserved residues within a peptide of defined length defines a motif for an immunogenic peptide. These residues are typically in close contact with the peptide binding groove, with their side chains buried in specific pockets of the groove itself. Typically, an immunogenic peptide will comprise up to three conserved residues, more usually two conserved residues.

As used herein, "negative binding residues" are amino acids which if present at certain positions will result in a peptide being a nonbinder or poor binder and in turn fail to induce a CTL response despite the presence of the appropriate conserved residues within the peptide.

The term "motif" refers to the pattern of residues in a peptide of defined length, usually about 8 to about 11 amino acids, which is recognized by a particular MHC allele. The peptide motifs are typically different for each human MHC allele and differ in the pattern of the highly conserved residues.

The binding motif for an allele can be defined with increasing degrees of precision. In one case, all of the conserved residues are present in the correct positions in a peptide and there are no negative binding residues present.

The phrases "isolated" or "biologically pure" refer to material which is substantially or essentially free from components which normally accompany it as found in its native state. Thus, the peptides of this invention do not contain materials normally associated with their in situ environment, e.g., MHC I molecules on antigen presenting cells. Even where a protein has been isolated to a homogenous or dominant band, there are trace contaminants in the range of 5-10% of native protein which co-purify with the desired protein. Isolated peptides of this invention do not contain such endogenous co-purified protein.

The term "residue" refers to an amino acid or amino acid mimetic incorporated in a oligopeptide by an amide bond or amide bond mimetic.

### DESCRIPTION OF THE PREFERRED EMBODIMENTS

The present invention is based on the determination of allele-specific peptide motifs for human Class I MHC (sometimes referred to as HLA) allele subtypes. These motifs were then used to define T cell epitopes from desired antigens, particularly those associated with human viral diseases, cancers or autoimmune diseases, for which the amino acid sequence of the potential antigen or autoantigen targets is known.

Epitopes on a number of potential target proteins were identified in this manner. Antigens included prostate specific antigen (PSA), hepatitis B core and surface antigens (HBVc, HBVs) hepatitis C antigens, Epstein-Barr virus antigens, melanoma antigens (e.g., MAGE-1), human immunodeficiency virus (HIV) antigens and human papilloma virus (HPV) antigens. The peptide of the invention is an epitope on HIV POL antigen.

Without wishing to be bound by theory, it is believed that the presentation of antigen by HLA Class I mediates suppression of autoreactive T cells by CD8⁺ suppressor T cells *(see, e.g.,* Jiang et al. Science 256:1213 (1992)). Such suppressor T cells release cytokines such as transforming growth factor-β (TGF-β), which specifically inhibit the autoreactive T cells. Miller et al. Proc. Natl. Acad. Sci. USA 89:421-425 (1992).

The peptide of the invention, SEQ ID NO:101, is synthesized and then tested for its ability to bind to the appropriate MHC molecules in assays using, for example, purified class I molecules and radioiodonated peptides and/or cells expressing empty class I molecules by, for instance, immunofluorescent staining and flow microfluorimetry, peptide-dependent class I assembly assays, and inhibition of CTL recognition by peptide competition. This peptide that binds to the class I Molecule is further evaluated for its ability to serve as target for CTLs derived from infected or immunized individuals, as well as for its capacity to induce primary in vitro or in vivo CTL responses that can give rise to CTL populations capable of reacting with virally infected target cells or tumor cells as potential therapeutic agents.

The MHC class I antigens are encoded by the HLA-A, B, and C loci. HLA-A and B antigens are expressed at the cell surface at approximately equal densities, whereas the expression of HLA-C is significantly lower (perhaps as much as 10-fold lower). Each of these loci have a number of alleles. The peptide binding motif identified herein is relatively specific for each allelic subtype.

For peptide-based vaccines, the peptide of the present invention comprises the motif recognized by an MHC I molecule of HLA-A loci A3.2 and A11 having a wide distribution in the human population. Since the MHC alleles occur at different frequencies within different ethnic groups and races, the choice of target MHC allele may depend upon the target population. Table 1 shows the frequency of various alleles at the HLA-A locus products among different races. For instance, the majority of the Caucasoid population can be covered by peptides which bind to four HLA-A allele subtypes, specifically HLA-A2.1, A1, A3.2, and A24.1. Similarly, the majority of the Asian population is encompassed with the addition of peptides binding to a fifth allele HLA-A 11.2.

**TABLE 1**

| A Allele/Subtype | N(69)* | A(54) | C(502) |
|---|---|---|---|
| A1 | 10.1(7) | 1.8(1) | 27.4(138) |
| A2.1 | 11.5(8) | 37.0(20) | 39.8(199) |
| A2.2 | 10.1(7) | 0 | 3.3(17) |
| A2.3 | 1.4(1) | 5.5(3) | 0.8(4) |
| A2.4 | - | - | - |
| A2.5 | - | - | - |
| A3.1 | 1.4(1) | 0 | 0.2(0) |
| A3.2 | 5.7(4) | 5.5(3) | 21.5(108) |
| A11.1 | 0 | 5.5(3) | 0 |
| A11.2 | 5.7(4) | 31.4(17) | 8.7(44) |
| A11.3 | 0 | 3.7(2) | 0 |
| A23 | 4.3(3) | - | 3.9(20) |
| A24 | 2.9(2) | 27.7(15) | 15.3(77) |
| A24.2 | - | - | - |
| A24.3 | - | - | - |
| A25 | 1.4(1) | - | 6.9(35) |
| A26.1 | 4.3(3) | 9.2(5) | 5.9(30) |
| A26.2 | 7.2(5) | - | 1.0(5) |
| A26V | - | 3.7(2) | - |
| A28.1 | 10.1(7) | - | 1.6(8) |
| A28.2 | 1.4(1) | - | 7.5(38) |
| A29.1 | 1.4(1) | - | 1.4(7) |
| A29.2 | 10.1(7) | 1.8(1) | 5.3(27) |
| A30.1 | 8.6(6) | - | 4.9(25) |
| A30.2 | 1.4(1) | - | 0.2(1) |
| A30.3 | 7.2(5) | - | 3.9(20) |
| A31 | 4.3(3) | 7.4(4) | 6.9(35) |
| A32 | 2.8(2) | - | 7.1(36) |
| Aw33.1 | 8.6(6) | - | 2.5(13) |
| Aw33.2 | 2.8(2) | 16.6(9) | 1.2(6) |
| Aw34.1 | 1.4(1) | - | - |
| Aw34.2 | 1.4.5(10) | - | 0.8(4) |
| Aw36 | 5.9(4) | - | - |

| | | | |
|---|---|---|---|
| Table compiled from B. DuPont, Immunobiology of HLA, Vol. I, Histocompatibility Testing 1987, Springer-Verlag, New York 1989. *N - negroid; A = Asian; C = caucasoid. Numbers in parenthesis represent the number of individuals included in the analysis. | | | |

The nomenclature used to describe peptide compounds follows the conventional practice wherein the amino group is presented to the left (the N-terminus) and the carboxyl group to the right (the C-terminus) of each amino acid residue. In the formulae representing selected specific embodiments of the present invention, the amino-and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids is represented by a lower case single letter or a lower case three letter symbol. Glycine has no asymmetric carbon atom and is simply referred to as "Gly" or G.

The procedures used to identify the peptide of the present invention generally followed the methods disclosed in Falk et al., Nature 351:290 (1991) Briefly, the methods involved large-scale isolation of MHC class I molecules, typically by immunoprecipitation or affinity chromatography, from the appropriate cell or cell line. Examples of other methods for isolation of the desired MHC molecule equally well known to the-artisan included ion exchange chromatography, lectin chromatography, size exclusion, high performance ligand chromatography, and a combination of all of the above techniques.

A large number of cells with defined MHC molecules, particularly MHC Class I molecules, are known and readily available. For example, human EBV-transformed B cell lines have been shown to be excellent sources for the preparative isolation of class I and class II MHC molecules. Well-characterized cell lines are available from private and commercial sources, such as American Type Culture Collection ("Catalogue of Cell Lines and Hybridomas," 6th edition (1988) Rockville, Maryland, U.S.A.); National Institute of General Medical Sciences 1990/1991 Catalog of Cell Lines (NIGMS) Human Genetic Mutant Cell Repository, Camden, NJ; and ASHI Repository, Bingham and Women's Hospital, 75 Francis Street, Boston, MA 02115. Table 2 list some B cell lines suitable for use as sources for HLA-A alleles. All of these cell lines can be grown in large batches and are therefore useful for large scale production of MHC molecules. One of skill will recognize that these are merely exemplary cell lines and that many other cell sources can be employed. Similar EBV B cell lines homozygous for HLA-B and HLA-C could serve as sources for HLA-B and HLA-C alleles, respectively.

**TABLE 2**

| HUMAN CELL LINES (HLA-A SOURCES) | |
|---|---|
| HLA-A allele | B cell line |
| A1 | MAT COX (9022) STEINLIN (9087) |
| A2.1 | JY |
| A3.2 | EHM (9080) HO301 (9055)GM3107 |
| A24.1 | KT3(9107),TISI (9042) |
| A11 | BVR (GM6828A) WT100 (GM8602)WT52 (GM8603) |

In the typical case, immunoprecipitation is used to isolate the desired allele. A number of protocols can be used, depending upon the specificity of the antibodies used. For example, allele-specific mAb reagents can be used for the affinity purification of the HLA-A, HLA-B, and HLA-C molecules. Several mAb reagents for the isolation of HLA-A molecules are available (Table 3). Thus, for each of the targeted HLA-A alleles, reagents are available that may be used for the direct isolation of the HLA-A molecules. Affinity columns prepared with these mAbs using standard techniques are successfully used to purify the respective HLA-A allele products.

In addition to allele-specific mAbs, broadly reactive anti-HLA-A, B, C mAbs, such as W6/32 and B9.12.1, and one anti-HLA-B, C mAb, B1.23.2, could be used in alternative affinity purification protocols as described in the example section below.

**TABLE 3**

| | |
|---|---|
| ANTIBODY REAGENTS | |
| anti-HLA | Name |
| HLA-A1 | 12/18 |
| HLA-A3 | GAPA3 (ATCC, HB122) |
| HLA-11,24.1 | A11.1M (ATCC, HB164) |
| HLA-A,B,C | W6/32 (ATCC, HB95) |
| monomorphic | B9.12.1 (INSERM-CNRS) |
| HLA-B,C | B.1.23.2 (INSERM-CNRS) |
| monomorphic | |

The peptides bound to the peptide binding groove of the isolated MHC molecules are eluted typically using acid treatment. Peptides can also be dissociated from class I molecules by a variety of standard denaturing means, such as heat, pH, detergents, salts, chaotropic agents, or a combination thereof.

Peptide fractions are further separated from the MHC molecules by reversed-phase high performance liquid chromatography (HPLC) and sequenced. Peptides can be separated by a variety of other standard means well known to the artisan, including filtration, ultrafiltration, electrophoresis, size chromatography, precipitation with specific antibodies, ion exchange chromatography, isoelectrofocusing, and the like.

Sequencing of the isolated peptides can be performed according to standard techniques such as Edman degradation (Hunkapiller, M.W., et al., Methods Enzymol. 91, 399 [1983]). Other methods suitable for sequencing include mass spectrometry sequencing of individual peptides as previously described (Hunt, et al., Science 225:1261 (1992), which is incorporated herein by reference). Amino acid sequencing of bulk heterogenous peptides (e.g., pooled HPLC fractions) from different class I molecules typically reveals a characteristic sequence motif for each class I allele.

Definition of motifs specific for different class I alleles allowed the identification of potential peptide epitopes from an antigenic protein whose amino acid sequence is known. Typically, identification of potential peptide epitopes was initially carried out using a computer to scan the amino acid sequence of a desired antigen for the presence of motifs. The epitopic sequences were then synthesized. The capacity to bind MHC Class molecules was measured in a variety of different ways. One means was a Class I molecular binding assay as described, for instance, in the related applications, noted above. Other alternatives described in the literature include inhibition of antigen presentation (Sette, et al., J. Immunol. 141:3893 (1991), in vitro assembly assays (Townsend, et al., Cell 62:285 (1990), and FACS based assays using mutated cells, such as RMA.S (Melief, et al., Eur. J. Immunol. 21:2963 [1991]).

Next, peptides that tested positive in the MHC class I binding assay were assayed for the ability of the peptides to induce specific CTL responses in vitro. For instance, antigen-presenting cells that had been incubated with a peptide were assayed for the ability to induce CTL responses in responder cell populations. Antigen-presenting cells can be normal cells such as peripheral blood mononuclear cells or dendritic cells (Inaba, et al., J. Exp. Med. 166:182 (1987); Boog, Eur. J. Immunol. 18:219 [1988]).

Alternatively, mutant mammalian cell lines that are deficient in their ability to load class I molecules with internally processed peptides, such as the mouse cell lines RMA-S (Kärre, et al.. Nature, 319:675 (1986); Ljunggren, et al., Eur. J. Immunol. 21:2963-2970 (1991)), and the human somatic T cell hybridoma, T-2 (Cerundolo, et al., Nature 345:449-452 (1990)) and which have been transfected with the appropriate human class I genes are conveniently used, when peptide is added to them, to test for the capacity of the peptide to induce in vitro primary CTL responses. Other eukaryotic cell lines which could be used include various insect cell lines such as mosquito larvae (ATCC cell lines CCL 125, 126, 1660, 1591, 6585, 6586), silkworm (ATTC CRL 8851), armyworm (ATCC CRL 1711), moth (ATCC CCL 80) and Drosophila cell lines such as a Schneider cell line (see Schneider J. Embryol. Exp. Morphol. 27:353-365 [1927]). That have been transfected with the appropriate human class I MHC allele encoding genes and the human B₂ microglobulin genes.

Peripheral blood lymphocytes are conveniently isolated following simple venipuncture or leukapheresis of normal donors or patients and used as the responder cell sources of CTL precursors. In one embodiment, the appropriate antigen-presenting cells are incubated with 10-100 µM of peptide in serum-free media for 4 hours under appropriate culture conditions. The peptide-loaded antigen-presenting cells are then incubated with the responder cell populations in vitro for 7 to 10 days under optimized culture conditions. Positive CTL activation can be determined by assaying the cultures for the presence of CTLs that kill radiolabeled target cells, both specific peptide-pulsed targets as well as target cells expressing endogenously processed form of the relevant virus or tumor antigen from which the peptide sequence was derived.

Specificity and MHC restriction of the CTL was determined by testing against different peptide target cells expressing appropriate or inappropriate human MHC class I. One peptide that tested positive in the MHC binding assays and gave rise to specific CTL responses is referred to herein as the immunogenic peptide consisting of SEQ ID NO:101.

The immunogenic peptide can be prepared synthetically, or by recombinant DNA technology or isolated from natural sources such as whole viruses or tumors. Although the peptide will preferably be substantially free of other naturally occurring host cell proteins and fragments thereof, in some embodiments the peptides can be synthetically conjugated to native fragments or particles. The peptide can be either in its neutral (uncharged) forms or in forms which are salts, and either free of modifications such as glycosylation, side chain oxidation, or phosphorylation or containing these modifications, subject to the condition that the modification not destroy the biological activity of the peptide as herein described.

In some embodiments it may be desirable to include in the pharmaceutical compositions of the invention at least one component which assists in priming CTL. Lipids have been identified as agents capable of assisting the priming CTL in vivo against viral antigens. For example, palmitic acid residues can be attached to the alpha and epsilon amino groups of a Lys residue and then linked, e.g., via one or more linking residues such as Gly, Gly-Gly-, Ser, Ser-Ser, or the like, to an immunogenic peptide. The lipidated peptide can then be injected directly in a micellar form, incorporated into a liposome or emulsified in an adjuvant, e.g., incomplete Freund's adjuvant. In a preferred embodiment a particularly effective immunogen comprises palmitic acid attached to alpha and epsilon amino groups of Lys, which is attached via linkage, e.g., Ser-Ser, to the amino terminus of the immunogenic peptide.

As another example of lipid priming of CTL responses, E. coli lipoproteins, such as tripalmitoyl-S-glycerylcysteinlyseryl-serine (P₃CSS) can be used to prime virus specific CTL when covalently attached to an appropriate peptide. See, Deres et al., Nature 342:561-564 (1989). The peptide of the invention can be coupled to P₃CSS, for example, and the lipopeptide administered to an individual to specifically prime a CTL response to the target antigen. Further, as the induction of neutralizing antibodies can also be primed with P₃CSS conjugated to a peptide which displays an appropriate epitope, the two compositions can be combined to more effectively elicit both humoral and cell-mediated responses to infection.

In addition, the peptide of the invention can be coupled to a carrier support. Modification at the C terminus in some cases may alter binding characteristics of the peptide. In addition, the peptide or oligopeptide sequences can differ from the natural sequence by being modified by terminal-NH₂ acylation, e.g., by alkanoyl (C₁-C₂₀) or thioglycolyl acetylation, terminal-carboxyl amidation, e.g., ammonia, methylamine, etc. In some instances these modifications may provide sites for linking to a support or other molecule.

The peptide of the invention can be prepared in a wide variety of ways. Because of its relatively short size, the peptide can be synthesized in solution or on a solid support in accordance with conventional techniques. Various automatic synthesizers are commercially available and can be used in accordance with known protocols. See, for example, Stewart and Young, Solid Phase Peptide Synthesis, 2d. ed., Pierce Chemical Co. (1984), supra.

Alternatively, recombinant DNA technology may be employed wherein a nucleotide sequence which encodes the immunogenic peptide of interest is inserted into an expression vector, transformed or transfected into an appropriate host cell and cultivated under conditions suitable for expression. These procedures are generally known in the art, as described generally in Sambrook et al., Molecular Cloning. A Laboratory Manual. Cold Spring Harbor Press, Cold Spring Harbor, New York (1982). Thus, fusion proteins which comprise a peptide sequence of the invention can be used to present the appropriate T cell epitope.

As the coding sequence for a peptide of the length contemplated herein can be synthesized by chemical techniques, for example, the phosphotriester method of Matteucci et al., J. Am. Chem._Soc. 103:3185 (1981), modification can be made simply by substituting the appropriate base(s) for those encoding the native peptide sequence. The coding sequence can then be provided with appropriate linkers and ligated into expression vectors commonly available in the art, and the vectors used to transform suitable hosts to produce the desired fusion protein. A number of such vectors and suitable host systems are now available. For expression of the fusion proteins, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts. Of course, yeast or mammalian cell hosts may also be used, employing suitable vectors and control sequences.

The peptide of the present invention and pharmaceutical and vaccine compositions thereof are useful for administration to mammals, particularly humans, to treat and/or prevent HIV infection. The disease which can be treated using the immunogenic peptide of the invention is AIDS.

For pharmaceutical compositions, the immunogenic peptide of the invention is administered to an individual already infected with HIV . Those in the incubation phase or the acute phase of infection can be treated with the immunogenic peptide separately or in conjunction with other treatments, as appropriate. In therapeutic applications, compositions are administered to a patient in an amount sufficient to elicit an effective CTL response to the HIV antigen and to cure or at least partially arrest symptoms and/or complications. An amount adequate to accomplish this is defined as "therapeutically effective dose." Amounts effective for this use will depend on, e.g., the peptide composition, the manner of administration, the stage and severity of the disease being treated, the weight and general state of health of the patient, and the judgment of the prescribing physician, but generally range for the initial immunization (that is for therapeutic or prophylactic administration) from about 1.0 µg to about 5000 µg of peptide for a 70 kg patient, followed by boosting dosages of from about 1.0 µg to about 1000 µg of peptide pursuant to a boosting regimen over weeks to months depending upon the patient's response and condition by measuring specific CTL activity in the patient's blood. It must be kept in mind that the peptide and compositions of the present invention may generally be employed in serious disease states, that is, life-threatening or potentially life threatening situations. In such cases, in view of the minimization of extraneous substances and the relative nontoxic nature of the peptide, it is possible and may be felt desirable by the treating physician to administer substantial excesses of these peptide compositions.

For therapeutic use, administration should begin at the first sign of HIV infection or shortly after diagnosis in the case of acute infection. This is followed by boosting doses until at least symptoms are substantially abated and for a period thereafter. In chronic infection, loading doses followed by boosting doses may be required.

Treatment of an infected individual with the compositions of the invention may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide compositions can also be used for the treatment of chronic infection and to stimulate the immune system to eliminate HIV infected cells in carriers. It is important to provide an amount of immuno-potentiating peptide in a formulation and mode of administration sufficient to effectively stimulate a cytotoxic T cell response. Thus, for treatment of chronic infection, a representative dose is in the range of about 1.0 µg to about 5000 µg, preferably about 5 µg to 1000 µg for a 7D kg patient per dose. Immunizing doses followed by boosting doses at established intervals, e.g., from one to four weeks, may be required, possibly for a prolonged period of time to effectively immunize an individual. In the case of chronic infection, administration should continue until at least clinical symptoms or laboratory tests indicate that the viral infection has been eliminated or substantially abated and for a period thereafter.

The pharmaceutical compositions for therapeutic treatment are intended for parenteral, topical, oral or local administration. Preferably, the pharmaceutical compositions are administered parenterally, e.g., intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier. A variety of aqueous carriers may be used, e.g., water, buffered water, 0.9% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of CTL stimulatory peptide of the invention in the pharmaceutical formulations can vary widely, i.e., from less than about 0.1 %, usually at or at least about 2% to as much as 20% to 50% or more by weight, and will be selected primarily by fluid volumes, viscosities, etc., in accordance with the particular mode of administration selected.

The peptide of the invention may also be administered via liposomes, which target the peptides to a particular cells tissue, such as lymphoid tissue. Liposomes are also useful in increasing the half-life of the peptides. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes filled with the peptide of the invention can be directed to the site of lymphoid cells, where the liposomes then deliver the therapeutic/immunogenic peptide compositions. Liposomes for use in the invention are formed from standard vesicle-forming lipids, which generally include neutral and negatively charged phospholipids and a sterol, such as cholesterol. The selection of lipids is generally guided by consideration of, e. g. , liposome size, acid lability and stability of the liposomes in the blood stream. A variety of methods are available for preparing liposomes, as described in, e.g., Szoka et al., Ann. Rev. Biophys.Bioeng. 9:467 (1980), U.S. Patent Nos. 4,235,871, 4,501,728, 4,837,028, and 5,019,369.

For targeting to the immune cells, a ligand to be incorporated into the liposome can include, e.g., antibodies or fragments thereof specific for cell surface determinants of the desired immune system cells. A liposome suspension containing the peptide may be administered intravenously, locally, topically, etc. in a dose which varies according to, inter alia, the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25 %-75 %.

For aerosol administration, the immunogenic peptide is preferably, supplied in finely divided form along with a surfactant and propellant. Typical percentages of peptides are 0.01 %-20% by weight, preferably 1 %-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactants may constitute 0.1 %-20 % by weight of the composition, preferably 0.25-5%. The balance of the composition is ordinarily propellant. A carrier can also be included, as desired, as with, e.g., lecithin for intranasal delivery.

In another aspect the present invention is directed to vaccines which contain as an active ingredient an immunogenically effective amount of the immunogenic peptide as described herein. The peptide may be introduced into a host, including humans, linked to its own carrier or as a homopolymer or heteropolymer of active peptide units. Such a polymer has the advantage of increased immunological reaction. Useful carriers are well known in the art, and include, e.g., thyroglobulin, albumins such as bovine serum albumin, tetanus toxoid, polyamino acids such as poly(lysine:glutamic acid), hepatitis B virus core protein, hepatitis B virus recombinant vaccine and the like. The vaccines can also contain a physiologically tolerable (acceptable) diluent such as water, phosphate buffered saline, or saline, and further typically include an adjuvant. Adjuvants such as incomplete Freund's adjuvant, aluminum phosphate, aluminum hydroxide, or alum are materials well known in the art. And, as mentioned above, CTL responses can be primed by conjugating peptides of the invention to lipids, such as P₃CSS. Upon immunization with a peptide composition as described herein, via injection, aerosol, oral, transdermal or other route, the immune system of the host responds to the vaccine by producing large amounts of CTLs specific for the antigen, and the host becomes at least partially immune to later infection, or resistant to developing chronic infection.

Vaccine compositions containing the peptide of the invention are administered to a patient susceptible to or otherwise at risk of viral infection or cancer to elicit an immune response against the antigen and thus enhance the patient's own immune response capabilities. Such an amount is defined to be an "immunogenically effective dose." In this use, the precise amounts again depend on the patient's state of health and weight, the mode of administration, the nature of the formulation, etc., but generally range from about 1.0 µg to about 5000 µg per 70 kilogram patient, more commonly from about 10 ug to about 500 µg mg per 70 kg of body weight.

In some instances it may be desirable to combine the peptide vaccines of the invention with vaccines which induce neutralizing antibody responses to the virus of interest, particularly to viral envelope antigens.

For therapeutic or immunization purposes, the peptide of the invention can also be expressed by attenuated viral hosts, such as vaccinia or fowlpox. This approach involves the use of vaccinia virus as a vector to express nucleotide sequences that encode the peptides of the invention. Upon introduction into an acutely or chronically infected host or into a noninfected host, the recombinant vaccinia virus expresses the immunogenic peptide, and thereby elicits a host CTL response. Vaccinia vectors and methods useful in immunization protocols are described in, e.g.,U.S. Patent No. 4,722,848. Another vector is BCG (Bacille Calmette Guerin). BCG vectors are described in Stover et al. (Nature 351:456-460 (1991). A wide variety of other vectors useful for therapeutic administration or immunization of the peptide of the invention, e.g., Salmonella typhi vectors and the like, will be apparent to those skilled in the art from the description herein.

Nucleic acids encoding the peptide of the invention can also be administered to the patient. This approach is described, for instance, in Wolff et. al., Science 247: 1465-1468 (1990) as well as U.S. Patent Nos. 5,580,859 and 5,589,466.

A preferred means of administering nucleic acids encoding the peptide of the invention uses minigene constructs encoding multiple epitopes of the invention. To create a DNA sequence encoding the selected CTL epitopes (minigene) for expression in human cells, the amino acid sequence of the epitope is reverse translated. A human codon usage table is used to guide the codon choice for each amino acid. These epitope-encoding DNA sequences are directly adjoined, creating a continuous polypeptide sequence. To optimize expression and/or immunogenicity, additional elements can be incorporated into the minigene design. Examples of amino acid sequence that could be reverse translated and included in the minigene sequence include: helper T lymphocyte epitopes, a leader (signal) sequence, and an endoplasmic reticulum retention signal. In addition, MHC presentation of CTL epitopes may be improved by including synthetic (e.g. poly-alanine) or naturally-occurring flanking sequences adjacent to the CTL epitopes.

The minigene sequence is converted to DNA by assembling oligonucleotides that encode the plus and minus strands of the minigene. Overlapping oligonucleotides (30-100 bases long) are synthesized, phosphorylated, purified and annealed under appropriate conditions using well known techniques. The ends of the oligonucleotides are joined using T4 DNA ligase. This synthetic minigene, encoding the CTL epitope polypeptide, can then cloned into a desired expression vector.

Standard regulatory sequences well known to those of skill in the art are included in the vector to ensure expression in the target cells. Several vector elements are required: a promoter with a down-stream cloning site for minigene insertion; a polyadenylation signal for efficient transcription termination; an *E*. *coli* origin of replication; and an *E*. *coli* selectable marker (e.g. ampicillin or kanamycin resistance). Numerous promoters can be used for this purpose, e.g., the human cytomegalovirus (hCMV) promoter. *See,* U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences can also be considered for increasing minigene expression. It has recently been proposed that immunostimulatory sequences (ISSs or CpGs) play a role in the immunogenicity of DNA vaccines. These sequences could be included in the vector, outside the minigene coding sequence, if found to enhance immunogenicity.

In some embodiments, a bicistronic expression vector, to allow production of the minigene-encoded epitopes and a second protein included to enhance or decrease immunogenicity can be used. Examples of proteins or polypeptides that could beneficially enhance the immune response if co-expressed include cytokines (e.g., IL2, IL12, GM-CSF), cytokine-inducing molecules (e.g. LeIF) or costimulatory molecules. Helper (HTL) epitopes could be joined to intracellular targeting signals and expressed separately from the CTL epitopes. This would allow direction of the HTL epitopes to a cell compartment different than the CTL epitopes. If required, this could facilitate more efficient entry of HTL epitopes into the MHC class II pathway, thereby improving CTL induction. In contrast to CTL induction, specifically decreasing the immune response by co-expression of immunosuppressive molecules (e.g. TGF-β) may be beneficial in certain diseases.

Once an expression vector is selected, the minigene is cloned into the polylinker region downstream of the promoter. This plasmid is transformed into an appropriate E. *coli* strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

Therapeutic quantities of plasmid DNA are produced by fermentation in *E*. *coli,* followed by purification. Aliquots from the working cell bank are used to inoculate fermentation medium (such as Terrific Broth), and grown to saturation in shaker flasks or a bioreactor according to well known techniques. Plasmid DNA can be purified using standard bioseparation technologies such as solid phase anion-exchange resins supplied by Quiagen. If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). This approach, known as "naked DNA," is currently being used for intramuscular (IM) administration in clinical trials. To maximize the immunotherapeutic effects of minigene DNA vaccines, an alternative method for formulating purified plasmid DNA may be desirable. A variety of methods have been described, and new techniques may become available. Cationic lipids can also be used in the formulation *(see, e.g.,* as described by Debs and Zhu (1993) WO 93/24640; Mannino and Gould-Fogerite (1988) BioTechniques 6(7): 682-691; Rose U.S. Pat No. 5,279,833; Brigham (1991) WO 91/06309; and Felgner et al. (1987) Proc. Natl. Acad. Sci. USA 84: 7413-7414). In addition, glycolipids, fusogenic liposomes, peptides and compounds referred to collectively as protective, interactive, non-condensing (PINC) could also be complexed to purified plasmid DNA to influence variables such as stability, intramuscular dispersion, or trafficking to specific organs or cell types.

The nucleic acids can also be administered using ballistic delivery as described, for instance, in U.S. Patent No. 5,204,253. Particles comprised solely of DNA can be administered. Alternatively, DNA can be adhered to particles, such as gold particles.

Target cell sensitization can be used as a functional assay for expression and MHC class I presentation of minigene-encoded CTL epitopes. The plasmid DNA is introduced into a mammalian cell line that is suitable as a target for standard CTL chromium release assays. The transfection method used will be dependent on the final formulation. Electroporation can be used for "naked" DNA, whereas cationic lipids allow direct *in vitro* transfection. A plasmid expressing green fluorescent protein (GFP) can be co-transfected to allow enrichment of transfected cells using fluorescence activated cell sorting (FACS). These cells are then chromium-51 labeled and used as target cells for epitope-specific CTL lines. Cytolysis, detected by 51Cr release, indicates production of MHC presentation of minigene-encoded CTL epitopes.

*In vivo* immunogenicity is a second approach for functional testing of minigene DNA formulations. Transgenic mice expressing appropriate human MHC molecules are immunized with the DNA product. The dose and route of administration are formulation dependent (e.g. IM for DNA in PBS, IP for lipid-complexed DNA). Twenty-one days after immunization, splenocytes are harvested and restimulated for 1 week in the presence of peptides encoding each epitope being tested. These effector cells (CTLs) are assayed for cytolysis of peptide-loaded, chromium-51 labeled target cells using standard techniques. Lysis of target cells sensitized by MHC loading of peptide corresponding to the minigene-encoded epitope demonstrates DNA vaccine function for in *vivo* induction of CTLs.

The antigenic peptide may be used to elicit CTL ex vivo, as well. The resulting CTL, can be used to treat chronic infections (viral or bacterial) or tumors in patients that do not respond to other conventional forms of therapy, or will not respond to a peptide vaccine approach of therapy. Ex vivo CTL responses to the pathogen (HIV) are induced by incubating in tissue culture the patient's CTL precursor cells (CTLp) together with a source of antigen-presenting cells (APC) and the immunogenic peptide. After an appropriate incubation time (typically 1-4 weeks), in which the CTLp are activated and mature and expand into effector CTL, the cells are infused back into the patient, where they will destroy their specific target cell (an infected cell or a tumor cell). In order to optimize the *in vitro* conditions for the generation of specific cytotoxic T cells, the culture of stimulator cells is maintained in an appropriate serum-free medium.

Prior to incubation of the stimulator cells with the cells to be activated, e.g., precursor CD8+ cells, an amount of antigenic peptide is added to the stimulator cell culture, of sufficient quantity to become loaded onto the human Class I molecules to be expressed on the surface of the stimulator cells. In the present invention, a sufficient amount of peptide is an amount that will allow about 200, and preferably 200 or more, human Class I MHC molecules loaded with peptide to be expressed on the surface of each stimulator cell. Preferably, the stimulator cells are incubated with > 20µg/ml peptide.

Resting or precursor CD8+ cells are then incubated in culture with the appropriate stimulator cells for a time period sufficient to activate the CD8+ cells. Preferably, the CD8+ cells are activated in an antigen-specific manner. The ratio of resting or precursor CD8+ (effector) cells to stimulator cells may vary from individual to individual and may further depend upon variables such as the amenability of an individual's lymphocytes to culturing conditions and the nature and severity of the disease condition or other condition for which the within-described treatment modality is used. Preferably, however, the lymphocyte:stimulator cell ratio is in the range of about 30:1 to 300:1. The effector/stimulator culture may be maintained for as long a time as is necessary to stimulate a therapeutically useable or effective number of CD8+ cells.

The induction of CTL *in vitro* requires the specific recognition of the peptide that is bound to allele specific MHC class I molecules on APC. The number of specific MHC/peptide complexes per APC is crucial for the stimulation of CTL, particularly in primary immune responses. While small amounts of peptide/MHC complexes per cell are sufficient to render a cell susceptible to lysis by CTL, or to stimulate a secondary CTL response, the successful activation of a CTL precursor (pCTL) during primary response requires a significantly higher number of MHC/peptide complexes. Peptide loading of empty major histocompatability complex molecules on cells allows the induction of primary cytotoxic T lymphocyte responses. Peptide loading of empty major histocompatability complex molecules on cells enables the induction of primary cytotoxic T lymphocyte responses.

Since mutant cell lines do not exist for every human MHC allele, it is advantageous to use a technique to remove endogenous MHC-associated peptides from the surface of APC, followed by loading the resulting empty MHC molecules with the immunogenic peptides of interest. The use of non-transformed (non-tumorigenic), non-infected cells, and preferably, autologous cells of patients as APC is desirable for the design of CTL induction protocols directed towards development of *ex vivo* CTL therapies. This application discloses methods for stripping the endogenous MHC-associated peptides from the surface of APC followed by the loading of desired peptides.

A stable MHC class I molecule is a trimeric complex formed of the following elements: 1) a peptide usually of 8 - 10 residues, 2) a transmembrane heavy polymorphic protein chain which bears the peptide-binding site in its α1 and α2 domains, and 3) a non-covalently associated non-polymorphic light chain, β₂microglobulin. Removing the bound peptides and/or dissociating the β₂microglobulin from the complex renders the MHC class I molecules nonfunctional and unstable, resulting in rapid degradation. All MHC class I molecules isolated from PBMCs have endogenous peptides bound to them. Therefore, the first step is to remove all endogenous peptides bound to MHC class I molecules on the APC without causing their degradation before exogenous peptides can be added to them.

Two possible ways to free up MHC class I molecules of bound peptides include lowering the culture temperature from 37°C to 26°C overnight to destablize β₂microglobulin and stripping the endogenous peptides from the cell using a mild acid treatment. The methods release previously bound peptides into the extracellular environment allowing new exogenous peptides to bind to the empty class I molecules. The cold-temperature incubation method enables exogenous peptides to bind efficiently to the MHC complex, but requires an overnight incubation at 26°C which may slow the cell's metabolic rate. It is also likely that cells not actively synthesizing MHC molecules (e.g., resting PBMC) would not produce high amounts of empty surface MHC molecules by the cold temperature procedure.

Harsh acid stripping involves extraction of the peptides with trifluoroacetic acid, pH 2, or acid denaturation of the immunoaffinity purified class I-peptide complexes. These methods are not feasible for CTL induction, since it is important to remove the endogenous peptides while preserving APC viability and an optimal metabolic state which is critical for antigen presentation. Mild acid solutions of pH 3 such as glycine or citrate-phosphate buffers have been used to identify endogenous peptides and to identify tumor associated T cell epitopes. The treatment is especially effective, in that only the MHC class I molecules are destabilized (and associated peptides released), while other surface antigens remain intact, including MHC class II molecules. Most importantly, treatment of cells with the mild acid solutions do not affect the cell's viability or metabolic state. The mild acid treatment is rapid since the stripping of the endogenous peptides occurs in two minutes at 4°C and the APC is ready to perform its function after the appropriate peptides are loaded. The technique is utilized herein to make peptide-specific APCs for the generation of primary antigen-specific CTL. The resulting APC are efficient in inducing peptide-specific CD8+ CTL.

Activated CD8+ cells may be effectively separated from the stimulator cells using one of a variety of known methods. For example, monoclonal antibodies specific for the stimulator cells, for the peptides loaded onto the stimulator cells, or for the CD8+ cells (or a segment thereof) may be utilized to bind their appropriate complementary ligand. Antibody-tagged molecules may then be extracted from the stimulator-effector cell admixture via appropriate means, e.g., via well-known immunoprecipitation or iminunoassay methods.

Effective, cytotoxic amounts of the activated CD8+ cells can vary between *in vitro* and *in vivo* uses, as well as with the amount and type of cells that are the ultimate target of these killer cells. The amount will also vary depending on the condition of the patient and should be determined via consideration of all appropriate factors by the practitioner. Preferably, however, about 1 X 10⁶ to about 1 X 10¹², more preferably about 1 X 10⁸ to about 1 X 10¹¹, and even more preferably, about 1 X 10⁹ to about 1 X 10¹⁰ activated CD8+ cells are utilized for adult humans, compared to about 5 X 10⁶ - 5 X 10⁷ cells used in mice.

Preferably, as discussed above, the activated CD8+ cells are harvested from the cell culture prior to administration of the CD8+ cells to the individual being treated. It is important to note, however, that unlike other present and proposed treatment modalities, the present method uses a cell culture system that is not tumorigenic. Therefore, if complete separation of stimulator cells and activated CD8+ cells is not achieved, there is no inherent danger known to be associated with the administration of a small number of stimulator cells, whereas administration of mammalian tumor-promoting cells may be extremely hazardous.

Methods of re-introducing cellular components are known in the art and include procedures such as those exemplified in U.S. Patent No. 4,844,893 to Honsik, et al. and U.S. Patent No. 4,690,915 to Rosenberg. For example, administration of activated CD8+ cells via intravenous infusion is appropriate.

The peptides may also find use as diagnostic reagents. For example, a peptide of the invention may be used to determine the susceptibility of a particular individual to a treatment regimen which employs the peptide or related peptides, and thus may be helpful in modifying an existing treatment protocol or in determining a prognosis for an affected individual. In addition, the peptides may also be used to predict which individuals will be at substantial risk for developing chronic infection.

To identify the peptide of the invention, class I antigen isolation, and isolation and sequencing of naturally processed peptides was carried out as described in the related applications. These peptides were then used to define specific binding motifs for each of the following alleles A3.2, A1, A11, and A24.1. These motifs are described in the related applications and summarized in Tables 5-8, below.

**TABLE 5**

| Summary | |
|---|---|
| HLA-A3.2 Allele-Specific Motif | |
| Position | Conserved Residues |
| 1 | - |
| 2 | V,L,M |
| 3 | Y,D |
| 4 | - |
| 5 | - |
| 6 | - |
| 7 | I |
| 8 | Q,N |
| 9 | K |
| 10 | K |

**TABLE 6**

| Summary | |
|---|---|
| HI A-A1 Allele-Specific Motif | |
| Position | Conserved Residues |
| 1 | - |
| 2 | S, T |
| 3 | D,E |
| 4 | P |
| 5 | - |
| 6 | - |
| 7 | L |
| 8 | - |
| 9 | Y |

**TABLE 7**

| Summary | |
|---|---|
| LA-A 11 Allele-Specific Motif | |
| Position | Conserved Residues |
| 1 | - |
| 2 | T,V |
| 3 | M,F |
| 4 | - |
| 5 | - |
| 6 | - |
| 7 | - |
| 8 | Q |
| 9 | K |
| 10 | K |

**Table 8**

| Summary | |
|---|---|
| HLA-A24.1 Allele-Specific Motif | |
| Position | Conserved Residues |
| 1 | - |
| 2 | Y |
| 3 | I,M |
| 4 | D,E,G,K,P |
| 5 | L,M,N |
| 6 | V |
| 7 | N,V |
| 8 | A,E,K,Q,S |
| 9 | F,L |
| 10 | F,A |

### Example 1

### Identification of immunogenic peptides

Using the motif identified above for various MHC class I allele amino acid sequences from various viral and tumor-related proteins were analyzed for the presence of these motifs. Screening was carried out described in the related applications. Table 9 provides the results of searches of the antigens.

**Table 9**

| SEQ. ID. No. | AA | SEQUENCE | SOURCE | ORJGIN_OR MOTIF |
|---|---|---|---|---|
| 1 | 9 | HSNLNDTTY | FLU A NP 140 | A03 |
| 2 | 9 | FVEALFQEY | P. falciparum CSP | A03/A11 |
| 3 | 9 | AADAAAAAY | A1 poly-A | A01 |
| 4 | 9 | DADAAAAAY | A1 poly-A | A01 |
| 5 | 9 | AADSAAAAY | A1 poly-A | A01 |
| 6 | 9 | AADAPAAAY | A1 poly-A | A01 |
| 7 | 9 | AADAAAQAY | A1 poly-A | A01 |
| 8 | 9 | QADAAAAAY | A1 poly-A | A01 |
| 9 | 9 | AADADAAAY | A1 poly-A | A01 |
| 10 | 9 | AADAOAAAY | A1 poly-A | A01 |
| 11 | 9 | AADAAKAAY | A1 poly-A | A01 |
| 12 | 9 | AADAAAPAY | A1 poly-A | A01 |
| 13 | 9 | AADAAAAPY | A1 poly-A | A01 |
| 14 | 9 | ATAAAAAAY | A1 poly-A | A01 |
| 15 | 9 | DTAAAAAAY | A1 poly-A | A01 |
| 16 | 9 | ATASAAAAY | A1 poly-A | A01 |
| 17 | 9 | ATAAPAAAY | A1 poly-A | A01 |
| 18 | 9 | ATAAAAQAY | A1 poly-A | A01 |
| 19 | 9 | ATAAAAAKY | A1 poly-A | A01 |
| 20 | 9 | QTAAAAAAY | A1 poly-A | A01 |
| 21 | 9 | ATAADAAAY | A1 poly-A | A01 |
| 22 | 9 | ATAAGAAAY | A1 poly-A | A01 |
| 23 | 9 | ATAAAKAAY | A1 poly-A | A01 |
| 24 | 9 | ATAAAAPAY | A1 poly-A | A01 |
| 25 | 9 | ATAAAAAPY | A1 poly-A | A01 |
| 26 | 9 | TYLISSIPL | GCDFP-15 70 | A24 |
| 27 | 9 | FYTNRTVQI | CCDFP-15 102 | A24 |
| 28 | 10 | PLQGAFNYKY | GCDFP-15 77 | A01 |
| 29 | 10 | LCDDNPKTFY | GCDFP-15 90 | A01 |
| 30 | 9 | TTNLRPTTY | GAD 21 | A01 |
| 31 | 9 | CLELAEYLY | GAD 483 | A01 |
| 321 | 9 | LLSPRPISY | HCV NS3 1157 | A01 |
| 33 | 9 | LLSPRPVSY | HCV NS3 1157 | A01 |
| 34 | 9 | PTVTVFHVY | HSV-1 POL 142 | A01 |
| 35 | 9 | ETAGRHVGY | HSV-1 POL 688 | A01 |
| 36 | 9 | GSGPELLFY | NSV-1 TERM 612 | A01 |
| 37 | 9 | LSPQWVADY | HSV-1 ENV 143 | A01 |
| 38 | 9 | VVERTDVYY | HSV-1 POL 252 | A01 |
| 39 | 9 | SLEHTLCTY | HSV-1 ENV 587 | A01 |
| 40 | 9 | PSQRHGSKY | Hu MBP 6 | A01 |
| 41 | 9 | CSAVPVYIY | Hu PLP 169 | A01 |
| 42 | 9 | LTFMIAATY | Hu PLP 255 | A01 |
| 43 | 9 | GTASFFFLY | Hu PLP 74 | A01 |
| 44 | 9 | GTEKLIETY | Hu PLP 42 | A01 |
| 45 | 9 | TTWCSQTSY | LCMV GP 217 | A01 |
| 46 | 9 | RTWENHCTY | LCMV GP 233 | A01 |
| 47 | 9 | QSSINISGY | LCMV NUC 232 | A01 |
| 48 | 9 | ITEMLRKDY | LCMV GP 417 | A01 |
| 49 | 9 | QSSFYSDWY | M. Tubere. 85A/3 | A01 |
| 50 | 9 | SSALTLAIY | M. Tubere. 85A/3 | A01 |
| 51 | 9 | ATWLGDDGY | M. Tubere. cat/p | A01 |
| 52 | 9 | QSTSINLPY | M. Tubere. DNAK | A01 |
| 53 | 9 | QSSFYSDWY | M. Tubere. 75 | A01 |
| 54 | 9 | YAELMTADY | M. Tubere. POL | A01 |
| 55 | 9 | STNEVTRIY | PSM 348 | A01 |
| 56 | 9 | RVDCTPLMY | PSM 463 | A01 |
| 57 | 9 | RGRRQPIPK | HCV CORE 59 | A03/A11 |
| 58 | 9 | KTKRNTNRR | HCV CORE 10 | A03/A11 |
| 59 | 9 | LGFGAYMSK | HCV NS3 1267 | A03/A11 |
| 60 | 9 | VAGALVAFK | HCV NS4 1864 | A03/A11 |
| 61 | 9 | NFISGIQYL | HCV NS4 1772 | A24 |
| 62 | 9 | FWAKHMWNF | HCV NS4 1765 | A24 |
| 63 | 10 | EVDGVRLHRY | HCV NS5 2129 | A01 |
| 64 | 10 | DLSGWFVAGY | HCV NS5 2999 | A01 |
| 65 | 10 | AACNWTRGER | HCV NSI/E2 647 | A03/A11 |
| 66 | 9 | KVYLAWVPA | HIV-I POL 74 | A03 |
| 67 | 9 | TLFCASDAK | HN-I ENV 82 | A03/A11 |
| 68 | 9 | ISLWDQSLK | HIV-1 ENV 78 | A03/A11 |
| 69 | 9 | RNELLGRR | HIV-1 ENV 53 | A03/A11 |
| 70 | 9 | MVHQAISPR | HIV-I GAG 45 | A03/A11 |
| 71 | 9 | TIKIGGQLK | HIV-I POL 65 | A03/A11 |
| 72 | 9 | KLVSAGIRK | HIV-I POL 57 | A03/A11 |
| 73 | 9 | KGLGISYGR | HIV-I TAT 77 | A03/A11 |
| 74 | 9 | GLGISYGRK | HIV-1 TAT 77 | A03/A11 |
| 75 | 9 | VMIVWQVDR | HIV-1 VIF 83 | A03/A11 |
| 76 | 9 | QMAVFIHNF | HIV-1 POL 92 | A03/A24 |
| 77 | 9 | SMTKILEPF | HIV-1 POL 87 | A03/A24 |
| 78 | 9 | TWGCSGKLI | HIV-1 ENV 69 | A24 |
| 79 | 9 | LYKYKVVKI | HIV-1 ENV 49 | A24 |
| 80 | 9 | VWKEATTTL | HIV-1 ENV 47 | A24 |
| 81 | 9 | GWMTNNPPI | HIV-1 GAG 31 | A24 |
| 82 | 9 | RFAVNPGLL | HIV-I GAG 26 | A24 |
| 83 | 9 | PYNTPVFA1 | HIV-1 POL 74 | A24 |
| 84 | 9 | WWAGIKQEF | HIV-1 POL 70 | A24 |
| 85 | 9 | LWQRPLVTI | HIV-1 POL 61 | A24 |
| 86 | 9 | IYETYGDTW | HIV-1 VPR 92 | A24 |
| 87 | 9 | PYNEWTLEL | HFV-1 VPR 56 | A24 |
| 88 | 10 | ILQQLLFlHF | HIV-1 VPR 72 | A03 |
| 89 | 10 | TTLFCASDAK | HIV-1 ENV 81 | A03/A11 |
| 90 | 10 | LLGTWGCSGK | HIV-1 ENV 73 | A03/A11 |
| 91 | 10 | IISLWDQSLK | HIV-1 ENV 66 | A03/A11 |
| 92 | 10 | LLQLTVWGIK | HIV-1 ENV 61 | A03/A11 |
| 93 | 10 | SILDIRQGPK | HIV-1 GAG 72 | A03/A11 |
| 94 | 10 | QMVHQAISPR | HIV-1 GAG 45 | A03/A11 |
| 95 | 10 | TAVQMAVFIH | HIV-1 POL 88 | A03/A11 |
| 96 | 10 | ISPIETVPVK | HIV-1 POL 87 | A03/A11 |
| 97 | 10 | LGIPHPAGLK | HIV-1 POL 87 | A03/A11 |
| 98 | 10 | PAIFQSSMTK | HIV-1 POL 78 | A03/A11 |
| 99 | 10 | KVYLAWVPAH | HIV-1 POL 74 | A03/A11 |
| 100 | 10 | DIIATDIQTK | HIV-1 POL 67 | A03/A11 |
| 101 | 10 | VTIKIGGQLK | HIV-1 POL 65 | A03/A11 |
| 102 | 10 | KAACWWAGIK | HIV-1 POL 65 | A03/A11 |
| 103 | 10 | VSQIIEQLIK | HIV-1 POL 61 | A03/A11 |
| 104 | 10 | KGLGISYGRK | HIV-1 TAT 77 | A03/A11 |
| 105 | 10 | VWKEATTTLF | HIV-1 ENV 47 | A24 |
| 106 | 10 | YWQATWIPEW | HIV-1 POL 96 | A24 |
| 107 | 10 | VYYDPSKDLI | HIV-1 POL 70 | A24 |
| 108 | | ALAAGAAAR | A3 poly-A | A03 |
| 109 | | AAAAGAAAK | A3 poly-A | A03 |
| 110 | 9 | AFLPWHRLF | Tyrosinase | A24 |
| 111 | 9 | AYGLDFYIL | p15 | A24 |

## Claims

1. An immunogenic peptide consisting of the amino acid sequence of SEQ ID NO: 101.

2. A composition comprising the immunogenic peptide of claim 1.

3. A composition of claim 2, further comprising a pharmaceutical acceptable carrier.

4. A composition according to claim 2, wherein the peptide is linked to a lipid.

5. A composition according to claim 2, wherein the peptide is linked to a carrier molecule.

6. A composition according to any of the preceding claims, wherein the peptide is incorporated as part of a liposome.

7. An expression vector comprising:
a nucleic acid sequence encoding an immunogenic peptide consisting of the amino acid sequence of SEQ ID NO. 101.

8. A peptide according to claim 1 or a composition according to any of claims 2 to 6 for use in medicine.

9. The use of a peptide according to claim 1 or of a composition according to any of claims 2 to 6 in the preparation of a medicament for treating HIV infection.

10. The use according to claim 9, wherein the medicament is a vaccine.

## Patentansprüche

1. Immunogenes Peptid bestehend aus der Aminosäuresequenz von SEQ ID NO: 101.

2. Zusammensetzung umfassend das immunogene Peptid von Anspruch 1.

3. Zusammensetzung nach Anspruch 2, ferner umfassend einen pharmazeutisch akzeptablen Träger.

4. Zusammensetzung nach Anspruch 2, wobei das Peptid mit einem Lipid verknüpft ist.

5. Zusammensetzung nach Anspruch 2, wobei das Peptid mit einer Trägermoleküle verknüpft ist.

6. Zusammensetzung nach irgendeinem der vorhergehenden Ansprüche, wobei das Peptid als Teil eines Liposoms aufgenommen wird.

7. Expressionvektor umfassend:
eine Nukleinsäuresequenz, die ein immunogenes Peptid kodiert, bestehend aus der Aminosäuresequenz von SEQ ID NO: 101.

8. Peptid nach Anspruch 1 oder eine Zusammensetzung nach irgendeinem der Ansprüche 2 bis 6 zur Anwendung in der Medizin.

9. Anwendung eines Peptids nach Anspruch 1 oder einer Zusammensetzung nach irgendeinem der Ansprüche 2 bis 6 in der Zubereitung eins Arzneimittels zur Behandlung von HIV-Infektion.

10. Anwendung nach Anspruch 9, wobei das Arzneimittel ein Impfstoff ist.

## Revendications

1. Peptide immunogénétique constitué de la séquence d'acide aminé SEQ ID NO: 101.

2. Composition comprenant le peptide immunogénétique de la revendication 1.

3. Composition selon la revendication 2, comprenant en outre un véhicule pharnaceutiquement acceptable.

4. Composition selon la revendication 2, dans laquelle le peptide est lié à un lipide.

5. Composition selon la revendication 2, dans laquelle le peptide est lié à une molécule véhicule.

6. Composition selon l'une quelconque des revendications précédentes, dans lequel le peptide est incorporé comme partie d'un liposome.

7. Vecteur d'expression comprenant:
une séquence d'acide nucléique encodant un peptide immunogénétique constitué de la séquence d'acide aminé SEQ ID NO: 101.

8. Peptide selon la revendication 1 ou composition selon l'une quelconque des revendications 2 à 6 en vue d'une utilisation médicale.

9. Utilisation d'un peptide selon la revendication 1 ou composition selon l'une quelconque des revendications 2 à 6 dans la préparation d'un médicament pour le traitement de l'infection HIV.

10. Utilisation selon la revendication 9, dans laquelle le médicament est un vaccin.
